# EUROPÄISCHE PATENTANMELDUNG

(11) **EP 4 837 605 A1**
(43) Veröffentlichungstag der Anmeldung: **11.11.2026**
(21) Anmeldenummer: 26175433.7
(22) Anmeldetag: 29.04.2026
(51) Int. Cl.: A61M 16/00

(54) **STEUEREINHEIT FÜR EIN BEATMUNGSGERÄT**

(30) Priorität: 05.05.2025 DE 102025001510
(71) Anmelder: Löwenstein Medical Technology S.A., 2557 Luxembourg (LU)
(72) Erfinder: Marschinke, Jörg, 56581 Ehlscheid (DE)
(74) Vertreter: Löwenstein Medical IP

(57) **Zusammenfassung**

Ein Beatmungsgerät (1) umfasst eine Aktorik (3) zum Erzeugen eines Atemluftstroms (5), einen Atemluftanschluss (7) zum Bereitstellen des Atemluftstroms (5) und eine Sensorik (15) zum Erfassen mindestens eines Beatmungsparameters (p, V) bezüglich einer Beatmung eines Patienten (2) mit dem vom Atemluftanschluss bereitgestellten Atemluftstrom, wobei der mindestens eine Beatmungsparameter einen am Atemluftanschluss anliegenden Atemluftdruck (p) und/oder ein vom Patienten ein- und/oder ausgeatmetes Atemluftvolumen (V) umfasst. Eine Steuereinheit (21) ist konfiguriert, um folgendes Steuerverfahren auszuführen: Empfangen von mittels der Sensorik erzeugten Sensordaten (17), die einen Istverlauf des mindestens einen Beatmungsparameters anzeigen; Erzeugen eines Steuersignals (26) zum Steuern der Aktorik unter Verwendung der Sensordaten, um den Atemluftstrom so anzupassen, dass sich der Istverlauf einem Sollverlauf annähert, wobei der Sollverlauf mehrere Stabilisierungsphasen, in denen sich der Istverlauf jeweils einer oberen Grenze annähern soll, und mehrere Entlastungsphasen, in denen sich der Istverlauf jeweils einer unteren Grenze annähern soll, umfasst, wobei die Stabilisierungsphasen und die Entlastungsphasen einander abwechseln, wobei die obere Grenze durch einen Referenzwert zum Stabilisieren der Lunge (13) des Patienten und zum Ermöglichen einer Spontanatmung des Patienten definiert ist und durch wiederholtes abwechselndes Erhöhen und Verringern ihres Werts relativ zum Referenzwert variiert wird, um in jeder Stabilisierungsphase eine zusätzliche Kohlendioxidabatmung zu bewirken, wobei die untere Grenze in ihrem Wert relativ zum Referenzwert so definiert ist, dass in jeder Entlastungsphase eine im Vergleich zur jeweils vorangegangenen Stabilisierungsphase verstärkte Ausatmung zum Entlasten der Lunge des Patienten bewirkt wird.

## Beschreibung

### Technisches Gebiet

Die Erfindung betrifft eine Steuereinheit für ein Beatmungsgerät. Des Weiteren betrifft die Erfindung ein durch eine solche Steuereinheit ausführbares Computerprogramm, ein entsprechendes computerlesbares Medium und ein Beatmungsgerät mit einer solchen Steuereinheit.

### Stand der Technik

Bei einer invasiven Beatmung eines Patienten kann der Beatmungsdruck beispielsweise so gesteuert werden, dass die Lunge des Patienten in mehreren Stabilisierungsphasen stabilisiert wird und gleichzeitig eine Spontanatmung des Patienten ermöglicht wird. Dabei können die Stabilisierungsphasen durch kürzere Entlastungsphasen unterbrochen werden, in denen der Beatmungsdruck deutlich niedriger als in der jeweils vorangegangenen Stabilisierungsphase ist, um eine entsprechend verstärkte Ausatmung zu bewirken (oder zu ermöglichen) und auf diese Weise die Lunge des Patienten vorübergehend zu entlasten. Ein derartiger Beatmungsmodus kann auch als Airway Pressure Release Ventilation, kurz APRV, bezeichnet werden. Bei der APRV kann es dazu kommen, dass der Patient in den Stabilisierungsphasen aufgrund einer verringerten Kohlendioxidabatmung mehr oder weniger sediert wird, ohne dass dem Patienten dazu Medikamente verabreicht zu werden brauchen. Eine solche kohlendioxidbedingte Sedierung oder Somnolenz kann unter bestimmten Umständen gewünscht sein, erfordert jedoch in der Regel eine engmaschige Überwachung des Patienten durch Pflegepersonal.

### Offenbarung der Erfindung

Eine Aufgabe der Erfindung kann darin gesehen werden, eine Steuereinheit bereitzustellen, die eine verbesserte Steuerung der Kohlendioxidabatmung beim Beatmen eines Patienten, beispielsweise bei einer invasiven APRV, und damit eine verbesserte Steuerung einer kohlendioxidbedingten Sedierung des Patienten ermöglicht. Eine weitere Aufgabe der Erfindung kann darin gesehen werden, ein entsprechendes Computerprogramm, ein entsprechendes computerlesbares Medium und ein entsprechendes Beatmungsgerät bereitzustellen.

Diese Aufgaben werden durch die Gegenstände der unabhängigen Ansprüche gelöst. Vorteilhafte Ausführungsformen sind in den abhängigen Ansprüchen, der nachfolgenden Beschreibung und den beigefügten Figuren dargelegt.

Ein erster Aspekt betrifft eine Steuereinheit für ein Beatmungsgerät.

Das Beatmungsgerät umfasst eine Aktorik zum Erzeugen eines Atemluftstroms, einen Atemluftanschluss zum Bereitstellen des Atemluftstroms und eine Sensorik zum Erfassen mindestens eines Beatmungsparameters bezüglich einer Beatmung eines Patienten mit dem vom Atemluftanschluss bereitgestellten Atemluftstrom. Der mindestens eine Beatmungsparameter umfasst einen am Atemluftanschluss (bei der Beatmung des Patienten) anliegenden Atemluftdruck und/oder ein vom Patienten (bei dessen Beatmung) ein- und/oder ausgeatmetes Atemluftvolumen.

Die Steuereinheit ist konfiguriert, um folgendes Steuerverfahren auszuführen: Empfangen von mittels der Sensorik erzeugten Sensordaten, die einen (beispielsweise zeitabhängigen) Istverlauf des mindestens einen Beatmungsparameters anzeigen; Erzeugen eines Steuersignals zum Steuern der Aktorik unter Verwendung der Sensordaten, um den Atemluftstrom so anzupassen, dass sich der Istverlauf einem Sollverlauf annähert. Der Sollverlauf umfasst mehrere Stabilisierungsphasen, in denen sich der Istverlauf jeweils einer oberen Grenze (für den jeweiligen Beatmungsparameter) annähern soll, und mehrere Entlastungsphasen, in denen sich der Istverlauf jeweils einer (unterhalb der oberen Grenze liegenden) unteren Grenze (für den jeweiligen Beatmungsparameter) annähern soll. Die Stabilisierungsphasen und die Entlastungsphasen wechseln einander ab. Die obere Grenze ist durch einen Referenzwert zum Stabilisieren der Lunge des Patienten und zum Ermöglichen einer Spontanatmung des Patienten definiert. Die obere Grenze wird ferner durch wiederholtes abwechselndes Erhöhen und Verringern ihres Werts relativ zum Referenzwert in jeder Stabilisierungsphase variiert, um in der jeweiligen Stabilisierungsphase eine zusätzliche Kohlendioxidabatmung zu bewirken. Die zusätzliche Kohlendioxidabatmung kann deutlich größer sein als eine normale Kohlendioxidabatmung bei einer konstanten oberen Grenze in der jeweiligen Stabilisierungsphase. Die untere Grenze ist in ihrem Wert relativ zum Referenzwert in jeder Entlastungsphase so definiert, dass in der jeweiligen Entlastungsphase eine im Vergleich zu einer der jeweiligen Entlastungsphase (unmittelbar) vorangegangenen Stabilisierungsphase verstärkte Ausatmung zum Entlasten der Lunge des Patienten bewirkt (oder zumindest ermöglicht) wird.

Eine derartige Steuereinheit ermöglicht es, die Kohlendioxidabatmung - und damit eine unter Umständen gewünschte kohlendioxidbedingte Sedierung oder Somnolenz des Patienten - in jeder Stabilisierungsphase gezielt, beispielsweise automatisch, zu steuern. Anders ausgedrückt ermöglicht eine derartige Steuereinheit eine gezielte Steuerung der Kohlendioxidabatmung - bei einer entsprechend angepassten APRV - während der gesamten Beatmungsdauer und nicht nur in den Entlastungsphasen. Somit können kurzfristig zu hohe Kohlendioxidkonzentrationen in der Atemluft und/oder im Blut des Patienten durch eine entsprechend eingestellte Abatmung wirksam verringert werden, ohne dass die Häufigkeit der Entlastungsphasen oder ein zeitliches Verhältnis zwischen Ein- und Ausatmungsphasen geändert zu werden braucht. In manchen Fällen kann dadurch sogar auf eine extrakorporale Membranoxygenierung, kurz ECMO, als möglicherweise letzte Therapiemaßnahme verzichtet werden. Ein weiterer Vorteil ist, dass aufgrund der gezielten Steuerung der Intensität der kohlendioxidbedingten Sedierung keine oder deutlich weniger sedierende Medikamente verabreicht werden können. Dadurch können unerwünschte Nebenwirkungen verhindert oder zumindest abgemildert werden.

Die Steuereinheit kann Mittel zur Datenverarbeitung umfassen. Die Mittel zur Datenverarbeitung können als Hard- und/oder Software implementiert sein und/oder einen Prozessor umfassen. Der Prozessor kann konfiguriert sein, um das (computerimplementierte) Steuerverfahren auszuführen. Zusätzlich zum Prozessor kann die Steuereinheit mindestens eines der folgenden Mittel zur Datenverarbeitung umfassen: einen Speicher, ein Bussystem zur Datenkommunikation zwischen dem Speicher und dem Prozessor, eine Datenkommunikationsschnittstelle zur drahtlosen und/oder drahtgebundenen Datenkommunikation mit Peripheriegeräten. Alternativ kann die Steuereinheit ausschließlich als Hardware, beispielsweise in Form eines ASIC- oder FPGA-Bausteins, implementiert sein.

Unter "Sensordaten" können vorzugsweise digitale Daten verstanden werden, die den Istverlauf in geeigneter Weise codieren. Bei den Sensordaten kann es sich beispielsweise um Ausgabedaten der Sensorik und/oder um verarbeitete Daten, die durch Verarbeiten eines (rohen, beispielsweise analogen) Ausgangssignals der Sensorik erzeugt wurden, handeln. Es ist möglich, dass die Sensordaten im Speicher der Steuereinheit (zwischen)gespeichert sind und von dort empfangen werden.

Unter "Istverlauf" kann ein gemessener und/oder geschätzter (beispielsweise zeitabhängiger) Verlauf des jeweiligen Beatmungsparameters verstanden werden. Anders ausgedrückt können die Sensordaten eine Zeitreihe von (gemessenen und/oder geschätzten) Istwerten des jeweiligen Beatmungsparameters als den Istverlauf umfassen.

In entsprechender Weise kann der Sollverlauf als eine Zeitreihe von (vorgegebenen) Sollwerten für den jeweiligen Beatmungsparameter aufgefasst werden. Der Sollverlauf kann beispielsweise in Form einer mathematischen Funktion und/oder einer Lookup-Tabelle im Speicher der Steuereinheit gespeichert sein.

Es ist möglich, dass jede Stabilisierungsphase unmittelbar in eine der Entlastungsphasen übergeht (oder umgekehrt). Zweckmäßigerweise kann jede Entlastungsphase deutlich kürzer als die jeweils vorangegangene Stabilisierungsphase sein. Jede Entlastungsphase kann beispielsweise nur eine einzige Ausatmung oder einen einzigen Atemzug und/oder nicht länger als 10 s, 5 s, 2 s oder 1 s dauern. Hingegen kann jede Stabilisierungsphase mehrere Atemzüge umfassen und/oder mindestens 2 s oder mindestens 4 s dauern. In manchen Fällen, beispielsweise wenn es sich bei dem Patienten um ein Neu- oder Frühgeborenes, einen Säugling oder ein Baby handelt, kann jede Entlastungsphase sogar deutlich kürzer als 1 s, vorzugsweise zwischen 0,3 s und 0,6 s, dauern. In diesen Fällen kann jede Stabilisierungsphase beispielsweise deutlich kürzer als 2 s dauern.

Die obere Grenze kann beispielsweise einem Solldruck, vorzugsweise einem vorgegebenen positiven endexspiratorischen Druck, und/oder einem Sollvolumen, vorzugsweise einem vorgegebenen Tidalvolumen, zum Stabilisieren der Lunge des Patienten und zum Ermöglichen einer Spontanatmung des Patienten entsprechen. Anders ausgedrückt hat der Patient aufgrund einer geeigneten oberen Grenze die Möglichkeit, in jeder Stabilisierungsphase spontan ein- und/oder auszuatmen, sofern er dazu in der Lage ist. Dabei kann die untere Grenze einem im Vergleich zur oberen Grenze deutlich niedrigeren Solldruck bzw. Sollvolumen zum Entlasten der Lunge des Patienten entsprechen. Die untere Grenze kann beispielsweise zwischen 10 % und 30 % der oberen Grenze (oder des Referenzwerts) betragen.

Das Variieren der oberen Grenze kann regelmäßig (beispielsweise entsprechend einer vorgegebenen Frequenz) und/oder unregelmäßig (beispielsweise abhängig von einer mehr oder weniger gleichmäßigen Spontanatmung des Patienten) erfolgen.

Es ist möglich, dass die obere Grenze beim Variieren ausgehend von ihrem jeweiligen aktuellen Wert einstufig oder - beispielsweise entsprechend einer geeigneten linearen oder nicht linearen mathematischen Funktion und/oder abhängig von einer jeweiligen Einatmungsdauer - mehrstufig erhöht und/oder verringert wird. Eine solche mathematische Funktion kann auch als Rampenfunktion oder kurz Rampe bezeichnet werden und/oder als Lookup-Tabelle im Speicher der Steuereinheit hinterlegt sein.

Zweckmäßigerweise kann die obere Grenze jeweils am Anfang und am Ende einer jeden Stabilisierungsphase (beispielsweise jeweils im ersten Zeitschritt und im letzten Zeitschritt einer jeden Stabilisierungsphase) auf den Referenzwert gesetzt werden.

Die obere Grenze kann auch als Atemplateau bezeichnet werden. Die untere Grenze kann je nach Beatmungsparameter auch als Entlastungsdruck oder Entlastungsvolumen bezeichnet werden.

Die untere Grenze kann in ihrem Wert relativ zum Referenzwert zusätzlich so definiert sein, dass auch in jeder Entlastungsphase eine Spontanatmung des Patienten ermöglicht wird. In jedem Fall sollte die untere Grenze so definiert sein, dass in jeder Entlastungsphase ein teilweiser oder vollständiger Kollaps der Lunge des Patienten vermieden wird.

Der Referenzwert kann beispielsweise einem Druck bzw. Volumen entsprechen, bei dem die Lunge des Patienten möglichst vollständig entfaltet ist. Der Referenzwert kann empirisch bestimmt worden sein, beispielsweise durch Einstellen des Referenzwerts unter Berücksichtigung einer (vom Referenzwert abhängigen) tatsächlichen Entfaltung der Lunge des Patienten bei dessen Beatmung mithilfe eines geeigneten bildgebenden Verfahrens (z. B. mithilfe einer Röntgenaufnahme).

Je nach Zustand des Patienten oder je nach Beatmungssituation kann der Referenzwert, durch den die obere Grenze in einer aktuellen Stabilisierungsphase definiert ist, vom Referenzwert, durch den die obere Grenze in mindestens einer früheren (d. h. der aktuellen Stabilisierungsphase vorangegangenen) Stabilisierungsphase definiert war, (deutlich) abweichen. Das Gleiche kann in entsprechender Weise für den Wert der unteren Grenze in einer aktuellen Entlastungsphase zutreffen.

Ein zweiter Aspekt betrifft ein Beatmungsgerät zum invasiven und/oder nicht invasiven Beatmen eines Patienten. Das Beatmungsgerät umfasst eine Aktorik zum Erzeugen eines Atemluftstroms, einen Atemluftanschluss zum Bereitstellen des Atemluftstroms, eine Sensorik zum Erfassen mindestens eines Beatmungsparameters bezüglich einer Beatmung des Patienten mit dem vom Atemluftanschluss bereitgestellten Atemluftstrom und eine Steuereinheit, wie sie vor- und nachstehend beschrieben wird. Der mindestens eine Beatmungsparameter umfasst einen am Atemluftanschluss (bei der Beatmung des Patienten) anliegenden Atemluftdruck und/oder ein vom Patienten (bei dessen Beatmung) ein- und/oder ausgeatmetes Atemluftvolumen.

Unter "Beatmungsgerät" kann auch ein Anästhesiegerät verstanden werden.

Der Atemluftanschluss kann über mindestens eine Atemluftleitung an den Patienten anschließbar ist, sodass eine Beatmung des Patienten mit dem Atemluftstrom möglich ist. Unter "Atemluftleitung" kann beispielsweise ein Beatmungsschlauch und/oder eine geeignete Patientenschnittstelle wie z. B. eine Maske, eine Nasenkanüle oder ein Tubus verstanden werden.

Die Aktorik kann einen oder mehrere elektropneumatische Aktoren zum Erzeugen des Atemluftstroms umfassen. Ein solcher Aktor kann beispielsweise ein Gebläse oder ein elektrisch steuerbares Ventil sein und/oder durch die Steuereinheit steuerbar sein.

Die Sensorik kann einen oder mehrere Sensoren zum Erfassen des jeweiligen Beatmungsparameters umfassen. Ein solcher Sensor kann beispielsweise ein Drucksensor zum Erfassen des Atemluftdrucks, ein Flusssensor zum Erfassen eines Volumenstroms einer vom Patienten ein- und/oder ausgeatmeten Atemluft oder ein Gassensor zum Erfassen eines Partialdrucks eines Atemgases (beispielsweise von Kohlendioxid oder Sauerstoff) in einer vom Patienten ein- und/oder ausgeatmeten Atemluft und/oder im Blut des Patienten sein. Je nach Ausführungsform kann ein solcher Sensor in einem Haupt- oder Nebenstrom der vom Patienten ein- und/oder ausgeatmeten Atemluft angeordnet sein.

Ein dritter Aspekt betrifft ein Computerprogramm zum Betreiben des vor- und nachstehend beschriebenen Beatmungsgeräts. Das Computerprogramm umfasst Befehle, die die Steuereinheit des Beatmungsgeräts - beispielsweise einen Prozessor der Steuereinheit - beim Ausführen des Computerprogramms durch die Steuereinheit (oder den Prozessor) veranlassen, das vor- und nachstehend beschriebene (computerimplementierte) Steuerverfahren auszuführen.

Ein vierter Aspekt betrifft ein computerlesbares Medium, auf dem das vor- und nachstehend beschriebene Computerprogramm gespeichert ist.

Das computerlesbare Medium kann ein flüchtiger oder nicht flüchtiger Datenspeicher sein. Beispielsweise kann das computerlesbare Medium eine Festplatte, ein USB-Speichergerät (USB = *universal serial bus*), ein RAM (*random-access memory*), ein ROM (*read-only memory*), ein EPROM (*erasable programmable read-only memory*)*,* ein EEPROM (*electrically erasable programmable read-only memory*), ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale der vor- und nachstehend beschriebenen Steuereinheit auch Merkmale des Computerprogramms und/oder des computerlesbaren Mediums sein können (und umgekehrt).

Im Folgenden werden verschiedene Ausführungsformen beschrieben. Diese Ausführungsformen sind nicht als Beschränkung des Umfangs der Offenbarung zu verstehen.

Gemäß einer Ausführungsform kann das Steuerverfahren ferner ein Auswerten der Sensordaten umfassen, um eine tatsächliche Spontanatmung des Patienten in einer der Stabilisierungsphasen zu erkennen. Alternativ kann das Steuerverfahren ferner umfassen: Empfangen von mittels der Sensorik erzeugten Volumenstromdaten, die einen (beispielsweise zeitabhängigen) Verlauf eines Volumenstroms einer vom Patienten (bei dessen Beatmung) ein- und/oder ausgeatmeten Atemluft anzeigen, und Auswerten der Volumenstromdaten allein oder in Kombination mit den Sensordaten, um eine tatsächliche Spontanatmung des Patienten in einer der Stabilisierungsphasen zu erkennen. In beiden Fällen kann - wenn die tatsächliche Spontanatmung erkannt wird - die obere Grenze in der jeweiligen Stabilisierungsphase abhängig von der erkannten tatsächlichen Spontanatmung, beispielsweise abhängig von einer Spontanatmungsfrequenz, mit der der Patient spontan atmet, variiert werden. Die tatsächliche Spontanatmung kann beispielsweise durch Vergleichen des Istverlaufs des mindestens einen Beatmungsparameters und/oder des Verlaufs des Volumenstroms mit einem entsprechenden (beispielsweise empirisch bestimmten) Referenzverlauf, der für eine spontane Ein- oder Ausatmung charakteristisch ist, erkannt werden.

Gemäß einer Ausführungsform kann - wenn eine spontane Einatmung als die tatsächliche Spontanatmung erkannt wird - die obere Grenze auf einen Spitzenwert gesetzt werden. Der Spitzenwert kann um einen vorgegebenen Betrag größer als der Referenzwert sein. Beispielsweise kann der Spitzenwert gleich einer Summe aus dem Referenzwert und einem Wert einer vorgegebenen Amplitude als dem vorgegebenen Betrag sein.

Zusätzlich oder alternativ kann - wenn eine spontane Ausatmung als die tatsächliche Spontanatmung erkannt wird - die obere Grenze auf einen Talwert gesetzt werden. Der Talwert kann gleich dem Referenzwert oder um einen vorgegebenen Betrag kleiner als der Referenzwert sein. Beispielsweise kann der Talwert gleich einer Differenz zwischen dem Spitzenwert und einem Wert einer vorgegebenen Amplitude als dem vorgegebenen Betrag sein.

Gemäß einer Ausführungsform kann die Steuereinheit zwischen einem ersten Variationsmodus und einem zweiten Variationsmodus umschaltbar sein. In diesem Fall ist es möglich, dass die obere Grenze - wenn der erste Variationsmodus aktiviert ist - abhängig von der Spontanatmung des Patienten und/oder - wenn der zweite Variationsmodus aktiviert ist - unabhängig von der Spontanatmung des Patienten variiert wird.

Beispielsweise kann die obere Grenze - wenn der erste Variationsmodus aktiviert ist - unter Berücksichtigung der erkannten tatsächlichen Spontanatmung (beispielsweise entsprechend einer erkannten Spontanatmungsfrequenz) und entsprechend einer vorgegebenen Amplitude variiert werden. Zusätzlich oder alternativ kann die obere Grenze - wenn der zweite Variationsmodus aktiviert ist - entsprechend einer vorgegebenen Frequenz oder entsprechend einer vorgegebenen Frequenz und einer vorgegebenen Amplitude variiert werden. Dabei kann eine etwaige erkannte tatsächliche Spontanatmung ignoriert werden.

Zweckmäßigerweise kann die Steuereinheit konfiguriert sein, um einen Betrieb im zweiten Variationsmodus zu verhindern, wenn der erste Variationsmodus aktiviert ist, und umgekehrt.

Der erste Variationsmodus kann beispielsweise einer Kombination eines APRV-Modus mit einem PSV-Modus (PSV = Pressure Support Ventilation; "druckunterstützte Spontanatmung") in jeder Stabilisierungsphase entsprechen.

Der zweite Variationsmodus kann beispielsweise einer Kombination eines APRV-Modus mit einem HFO-Modus (HFO = Hochfrequenzoszillation) in jeder Stabilisierungsphase entsprechen.

Gemäß einer Ausführungsform kann die obere Grenze entsprechend einer vorgegebenen Frequenz und/oder einer vorgegebenen Amplitude variiert werden.

Das Variieren der oberen Grenze entsprechend der vorgegebenen Amplitude kann beispielsweise ein Erhöhen der oberen Grenze durch Addieren eines Werts der vorgegebenen Amplitude zu einem aktuellen Wert der oberen Grenze (beispielsweise zum Referenz- oder Talwert) und/oder ein Verringern der oberen Grenze durch Subtrahieren eines Werts der vorgegebenen Amplitude von einem aktuellen Wert der oberen Grenze (beispielsweise vom Referenz- oder Spitzenwert) umfassen.

Beispielsweise kann die obere Grenze entweder entsprechend der vorgegebenen Frequenz oder entsprechend der vorgegebenen Frequenz und der vorgegebenen Amplitude variiert werden, solange (beispielsweise im ersten Variationsmodus) keine tatsächliche Spontanatmung des Patienten in der jeweiligen Stabilisierungsphase erkannt wird und/oder wenn der zweite Variationsmodus aktiviert ist.

Gemäß einer Ausführungsform kann das Steuerverfahren ferner umfassen: Empfangen von mittels der Sensorik erzeugten Partialdruckdaten, die einen oder mehrere Partialdruckwerte und/oder einen beispielsweise zeitabhängigen Verlauf eines Kohlendioxidpartialdrucks (und/oder eines Partialdrucks mindestens eines weiteren Atemgases wie beispielsweise Sauerstoff, Stickstoff oder Narkosegas) in einer vom Patienten ausgeatmeten Atemluft und/oder im Blut des Patienten umfassen; Bestimmen der vorgegebenen Frequenz unter Verwendung der Partialdruckdaten und einer Frequenzzuordnungsvorschrift, die möglichen Partialdruckwerten jeweils eine bestimmte Frequenz zuordnet. Zusätzlich oder alternativ kann das Steuerverfahren umfassen: Bestimmen der vorgegebenen Amplitude unter Verwendung der Partialdruckdaten und einer Amplitudenzuordnungsvorschrift, die möglichen Partialdruckwerten jeweils eine bestimmte Amplitude zuordnet.

Dies ermöglicht es, die Kohlendioxidabatmung in jeder Stabilisierungsphase automatisch abhängig von einem tatsächlichen Kohlendioxidpartialdruck in der vom Patienten ausgeatmeten Atemluft bzw. im Blut des Patienten zu steuern, beispielsweise indem die vorgegebene Frequenz bzw. Amplitude in ihrem Wert bei einem zu hohen tatsächlichen Kohlendioxidpartialdruck entsprechend erhöht wird, um eine entsprechend stärkere Kohlendioxidabatmung (und damit eine entsprechende Verringerung des tatsächlichen Kohlendioxidpartialdrucks) zu bewirken, und/oder bei einem zu niedrigen tatsächlichen Kohlendioxidpartialdruck entsprechend verringert wird, um eine entsprechend schwächere Kohlendioxidabatmung (und damit eine entsprechende Erhöhung des tatsächlichen Kohlendioxidpartialdrucks) zu bewirken. Mithilfe einer solchen Regelung kann der tatsächliche Kohlendioxidpartialdruck schnell und genau einem gewünschten Kohlendioxidpartialdruck angenähert werden. Dies hat den Vorteil, dass der Aufwand beim Überwachen des Patienten durch Pflegepersonal im Vergleich zu einer Ausführungsform ohne eine solche Regelung deutlich verringert werden kann.

Unter "Zuordnungsvorschrift" wie in "Frequenzzuordnungsvorschrift" oder "Amplitudenzuordnungsvorschrift" kann beispielsweise eine mathematische Funktion oder eine Lookup-Tabelle verstanden werden. Eine solche Zuordnungsvorschrift kann beispielsweise im Speicher der Steuereinheit hinterlegt sein. Je nach Partialdruckwert kann die vorgegebene Frequenz und/oder kann die vorgegebene Amplitude auch null betragen.

Gemäß einer Ausführungsform kann das Steuerverfahren ferner ein Bestimmen der vorgegebenen Frequenz und/oder der vorgegebenen Amplitude unter Berücksichtigung der Spontanatmung des Patienten, beispielsweise entsprechend einer Spontanatmungsfrequenz, mit der der Patient spontan atmet, umfassen.

Gemäß einer Ausführungsform kann die vorgegebene Frequenz zwischen 5 Hz und 25 Hz, vorzugsweise zwischen 10 Hz und 20 Hz, betragen. Ein solcher Frequenzbereich hat sich in der Praxis als besonders wirksam erwiesen.

Gemäß einer Ausführungsform kann die vorgegebene Amplitude einer Druckdifferenz zwischen 2 mbar und 15 mbar, vorzugsweise von mindestens 3 mbar und/oder höchstens 10 mbar, entsprechen. Ein solcher Druckdifferenzbereich hat sich in der Praxis als besonders wirksam erwiesen.

Gemäß einer Ausführungsform kann das Steuerverfahren ferner umfassen: Empfangen von mittels der Sensorik erzeugten Volumenstromdaten, die einen (beispielsweise zeitabhängigen) Verlauf eines Volumenstroms einer vom Patienten in einer aktuellen Entlastungsphase ausgeatmeten Atemluft anzeigen; Bestimmen eines Übergangszeitpunkts, zu dem die aktuelle Entlastungsphase in eine neue Stabilisierungsphase übergehen soll, unter Verwendung der Volumenstromdaten; Umschalten des Sollverlaufs von der unteren Grenze auf die obere Grenze zum Übergangszeitpunkt, sodass die aktuelle Entlastungsphase in die neue Stabilisierungsphase übergeht. Auf diese Weise kann eine vollständige Entleerung und somit ein teilweiser oder vollständiger Kollaps der Lunge des Patienten, insbesondere der Alveolen, beim Ausatmen in der aktuellen Entlastungsphase wirksam verhindert werden.

Anders ausgedrückt kann die Steuereinheit bzw. das Steuerverfahren - um den für die untere Grenze eingestellten Wert zusätzlich abzusichern - eine spezielle Flusssteuerungsfunktion umfassen, die sich an einem exspiratorischen Flussprofil orientiert und beispielsweise anhand einer geeigneten Prozentvorgabe den Volumenstrom oder Fluss steuert. Dies ermöglicht es, die Entlastungsphasen flexibel an plötzliche Änderungen von Compliance und Resistance während der Beatmung, beispielsweise infolge einer Umlagerung des Patienten, anzupassen.

Gemäß einer Ausführungsform kann der Verlauf des Volumenstroms mit einem Schwellenwert verglichen werden. Dabei kann ein Zeitpunkt, zu dem der Verlauf des Volumenstroms (erstmals) den Schwellenwert erreicht, als der Übergangszeitpunkt bestimmt werden. Der Schwellenwert kann beispielsweise einem Anteil zwischen 10 % und 70 % oder zwischen 20 % und 50 %, vorzugsweise 25 %, eines Maximums des Volumenstroms der vom Patienten bei einem einzelnen Atemzug ausgeatmeten Atemluft entsprechen.

Gemäß einer Ausführungsform kann die obere Grenze einem positiven endexspiratorischen Druck zwischen 5 mbar und 60 mbar, zwischen 5 mbar und 40 mbar, zwischen 5 mbar und 30 mbar, zwischen 5 mbar und 25 mbar oder zwischen 5 mbar und 20 mbar entsprechen. Dabei kann der Referenzwert um einen vorgegebenen Betrag, der beispielsweise zwischen 2 mbar und 15 mbar, vorzugsweise zwischen 3 mbar und 10 mbar, liegen kann, kleiner als der jeweils größte Wert der vorgenannten Wertebereiche sein. Beispielsweise kann der jeweils größte Wert dieser Wertebereiche einem größten zulässigen Wert und/oder dem Spitzenwert entsprechen.

Gemäß einer Ausführungsform kann die untere Grenze einem positiven endexspiratorischen Druck zwischen 0 mbar und 30 mbar oder zwischen 0 mbar und 5 mbar entsprechen. Zusätzlich oder alternativ kann die untere Grenze in jeder Entlastungsphase konstant sein.

Gemäß einer Ausführungsform kann ein zeitliches Verhältnis der Stabilisierungsphasen zu den Entlastungsphasen bezogen auf eine geeignete Referenzdauer mindestens zwei zu eins oder mindestens vier zu eins betragen. Anders ausgedrückt kann jede Stabilisierungsphase deutlich länger als die jeweils (unmittelbar) nachfolgende Entlastungsphase, beispielsweise mindestens zweimal oder mindestens viermal so lang wie die jeweils (unmittelbar) nachfolgende Entlastungsphase, sein.

Gemäß einer Ausführungsform kann jede Stabilisierungsphase mindestens zwei oder mindestens vier Atemzüge umfassen. Anders ausgedrückt kann jede Stabilisierungsphase mindestens so lang sein, dass mindestens zwei oder mindestens vier unmittelbar aufeinanderfolgende (spontane) Atemzüge in der jeweiligen Stabilisierungsphase möglich sind.

### Kurze Beschreibung der Zeichnungen

Im Folgenden werden beispielhafte Ausführungsformen mit Bezug auf die beigefügten Zeichnungen beschrieben. Weder die Beschreibung noch die Zeichnungen sind als Beschränkung des Umfangs der Offenbarung zu verstehen.

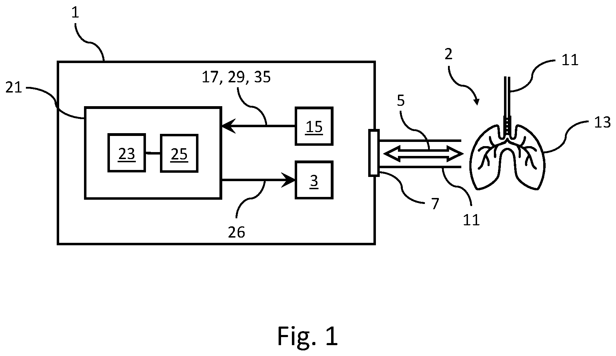

Fig. 1 zeigt eine beispielhafte Ausführungsform eines Beatmungsgeräts.

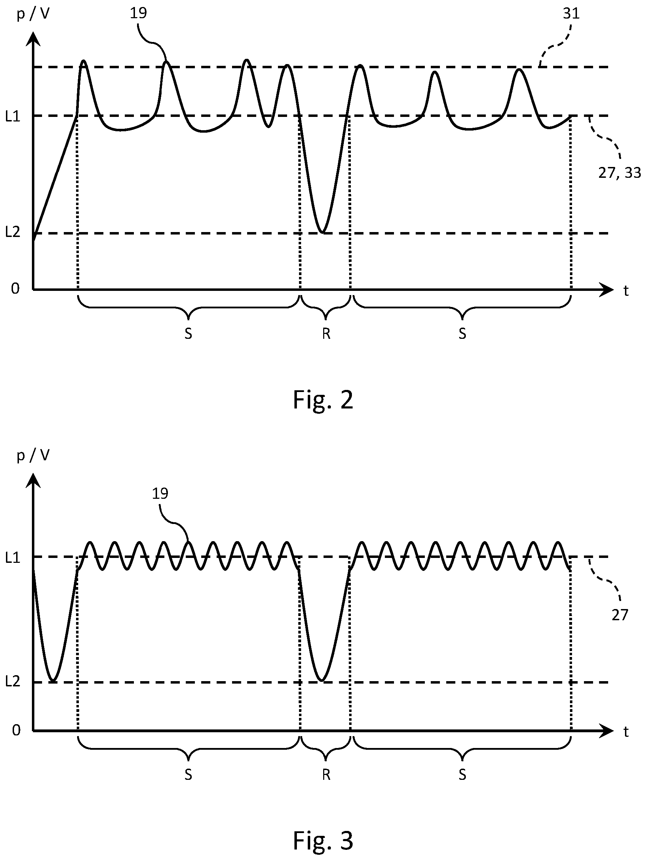

Fig. 2 zeigt einen zeitabhängigen Istverlauf von Beatmungsparametern, die durch eine Steuereinheit aus Fig. 1 entsprechend einem Sollverlauf mit sich abwechselnden Stabilisierungs- und Entlastungsphasen gesteuert werden können, wobei der Sollverlauf in jeder Stabilisierungsphase abhängig von einer Spontanatmung variiert wird.
Fig. 3 zeigt einen zeitabhängigen Istverlauf von Beatmungsparametern, die durch eine Steuereinheit aus Fig. 1 entsprechend einem Sollverlauf mit sich abwechselnden Stabilisierungs- und Entlastungsphasen gesteuert werden können, wobei eine von einer Spontanatmung unabhängige Hochfrequenzoszillation als der Sollverlauf in jeder Stabilisierungsphase verwendet wird.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen

Fig. 1 zeigt ein Beatmungsgerät 1 zum invasiven Beatmen eines Patienten 2. Das Beatmungsgerät 1 umfasst eine Aktorik 3 zum Erzeugen eines Atemluftstroms 5 und einen Atemluftanschluss 7 zum Bereitstellen des Atemluftstroms 5. Der Atemluftanschluss 7 ist über eine oder mehrere geeignete Atemluftleitungen 11, beispielsweise einen oder mehrere Beatmungsschläuche und einen Tubus, an den Atemapparat des Patienten 2, genauer an dessen Lunge 13, angeschlossen, sodass eine Beatmung des Patienten 2 mit dem Atemluftstrom 5 möglich ist.

Zusätzlich oder alternativ kann das Beatmungsgerät 1 zum nicht invasiven Beatmen des Patienten 2, beispielsweise über eine Mund-, Nasen- oder Gesichtsmaske oder eine Nasenkanüle, geeignet sein.

Die Aktorik 3 kann einen oder mehrere elektropneumatische Aktoren zum Erzeugen des Atemluftstroms 5 umfassen, beispielsweise ein oder mehrere Gebläse und/oder ein oder mehrere elektrisch steuerbare Ventile.

Darüber hinaus umfasst das Beatmungsgerät 1 eine Sensorik 15 zum Erfassen mindestens eines Beatmungsparameters bezüglich der Beatmung des Patienten 2, hier eines am Atemluftanschluss 7 anliegenden Atemluftdrucks p (siehe Fig. 2 und Fig. 3). Dementsprechend kann die Sensorik 15 einen oder mehrere Drucksensoren zum Erfassen des Atemluftdrucks p umfassen. Die Sensorik 15 ist ausgebildet, um entsprechende Sensordaten 17 zu erzeugen und auszugeben. Die Sensordaten 17 codieren in diesem Beispiel einen zeitabhängigen Istverlauf 19 des Atemluftdrucks p bei der Beatmung des Patienten 2.

Zusätzlich oder alternativ kann die Sensorik 15 ausgebildet sein, um ein vom Patienten 2 ein- und/oder ausgeatmetes Atemluftvolumen V (siehe Fig. 2 und Fig. 3) als den Beatmungsparameter zu erfassen. Dazu kann die Sensorik 15 einen oder mehrere Flusssensoren zum Erfassen eines Volumenstroms einer vom Patienten 2 ein- und/oder ausgeatmeten Atemluft umfassen. Das Atemluftvolumen V kann beispielsweise durch Integrieren des erfassten Volumenstroms bestimmt werden. Ein solcher Flusssensor kann in einem Haupt- oder Nebenstrom der vom Patienten 2 ein- und/oder ausgeatmeten Atemluft angeordnet sein.

Zudem umfasst das Beatmungsgerät 1 eine Steuereinheit 21 zum Steuern der Aktorik 3. Die Steuereinheit 21 kann einen Prozessor 23 und einen Speicher 25 umfassen. Im Speicher 25 kann ein Computerprogramm zum Betreiben des Beatmungsgeräts 1 gespeichert sein. Der Prozessor 23 kann konfiguriert sein, um durch Ausführen des Computerprogramms ein Steuerverfahren zum Steuern des Atemluftstroms 5 auszuführen. Das Steuerverfahren kann beispielsweise fortlaufend (und/oder in mehreren aufeinanderfolgenden Zeitschritten) während der Beatmung des Patienten 2 oder während des Betriebs des Beatmungsgeräts 1 ausgeführt werden.

In einem ersten Schritt des Steuerverfahrens werden die Sensordaten 17 empfangen.

In einem zweiten Schritt des Steuerverfahrens wird ein Steuersignal 26 zum Steuern der Aktorik 3 unter Verwendung der Sensordaten 17 erzeugt, um den Atemluftstrom 5 so anzupassen, dass sich der Istverlauf 19 einem Sollverlauf zwischen einer oberen Grenze L1 und einer unteren Grenze L2 für den jeweiligen Beatmungsparameter, hier den Atemluftdruck p, annähert. Je nach Ausführungsform kann sich der Sollverlauf auch auf das Atemluftvolumen V oder einen anderen geeigneten Beatmungsparameter beziehen.

Der Sollverlauf umfasst mehrere Stabilisierungsphasen S, in denen sich der Istverlauf 19 jeweils der oberen Grenze L1 annähern soll, und mehrere Entlastungsphasen R, in denen sich der Istverlauf 19 jeweils der unteren Grenze L2 annähern soll (in Fig. 2 und Fig. 3 wird beispielhaft jeweils nur eine der Entlastungsphasen R gezeigt). Die Stabilisierungsphasen S können mit den Entlastungsphasen R abwechseln. Dabei kann jede Stabilisierungsphase S unmittelbar in eine der Entlastungsphasen R übergehen und/oder jede Entlastungsphase R unmittelbar in eine der Stabilisierungsphasen S übergehen.

Die obere Grenze L1 ist durch einen Referenzwert 27 zum Stabilisieren der Lunge 13 des Patienten 2 und zum Ermöglichen einer Spontanatmung des Patienten 2 definiert. In diesem Beispiel ist der Referenzwert 27 ein geeigneter Druckwert, beispielsweise ein geeigneter Wert eines positiven endexspiratorischen Drucks (kurz PEEP).

Die untere Grenze L2 ist in ihrem Wert relativ zum Referenzwert 27 so definiert, dass in jeder Entlastungsphase R eine im Vergleich zur jeweils unmittelbar vorangegangenen Stabilisierungsphase S verstärkte Ausatmung zum Entlasten der Lunge 13 des Patienten 2 bewirkt (oder zumindest ermöglicht) wird. Dabei wird entsprechend viel Kohlendioxid abgeatmet. Die untere Grenze L2 kann so gewählt sein, dass auch in jeder Entlastungsphase R eine Spontanatmung des Patienten 2 ermöglicht wird. Generell kann die untere Grenze L2 betragsmäßig kleiner als die obere Grenze L1 sein. In diesem Beispiel entspricht die obere Grenze L1 einem höheren positiven endexspiratorischen Druck und die untere Grenze L2 einem im Vergleich zur oberen Grenze L1 deutlich niedrigeren positiven endexspiratorischen Druck.

Um die Kohlendioxidabatmung in den Stabilisierungsphasen S zu verbessern, wird die obere Grenze L1 in jeder Stabilisierungsphase S durch wiederholtes abwechselndes Erhöhen und Verringern ihres Werts relativ zum Referenzwert 27 variiert. Dies kann abhängig oder unabhängig von einer etwaigen Spontanatmung des Patienten 2 erfolgen.

Dabei kann ein zeitliches Verhältnis der Stabilisierungsphasen S zu den Entlastungsphasen R bezogen auf eine Referenzdauer mindestens zwei zu eins betragen, beispielsweise drei zu eins, vier zu eins, fünf zu eins, sechs zu eins oder mehr, in manchen Fällen sogar zehn zu eins (oder mehr). Die Referenzdauer kann beispielsweise 5 s, 10 s, 1 min oder zwischen 1 min und 10 min betragen oder gleich einer (durchschnittlichen) Dauer eines einzelnen Atemzugs oder mehrerer (unmittelbar) aufeinanderfolgender Atemzüge sein. Ein derartiges zeitliches Verhältnis kann beispielsweise vorgegeben sein oder aus einer (beispielsweise vorgegebenen) Häufigkeit, mit der die Stabilisierungsphasen S durch eine Entlastungsphase R unterbrochen werden sollen, resultieren.

Zweckmäßigerweise können die Stabilisierungsphasen S jeweils mindestens zwei oder mindestens vier (unmittelbar aufeinanderfolgende) Atemzüge umfassen. Möglich sind aber auch Stabilisierungsphasen S, die jeweils nur einen einzelnen Atemzug oder jeweils nur eine einzelne Ein- oder Ausatmungsphase eines einzelnen Atemzugs umfassen.

Zusätzlich können die Sensordaten 17 ausgewertet werden, um eine tatsächliche Spontanatmung des Patienten 2 in einer der Stabilisierungsphasen S zu erkennen.

Alternativ können mittels der Sensorik 15 (beispielsweise mittels des Flusssensors oder der Flusssensoren) erzeugte Volumenstromdaten 29 empfangen werden, die einen (beispielsweise zeitabhängigen) Verlauf eines Volumenstroms einer vom Patienten 2 ein- und/oder ausgeatmeten Atemluft anzeigen. In diesem Fall ist es möglich, dass die Volumenstromdaten 29 allein oder in Kombination mit den Sensordaten 17 ausgewertet werden, um eine tatsächliche Spontanatmung des Patienten 2 in einer der Stabilisierungsphasen S zu erkennen.

In beiden Fällen kann - wenn die tatsächliche Spontanatmung erkannt wird - die obere Grenze L1 in der jeweiligen Stabilisierungsphase S abhängig von der erkannten tatsächlichen Spontanatmung variiert werden (siehe Fig. 2), beispielsweise abhängig von einer Spontanatmungsfrequenz, mit der der Patient 2 spontan atmet, und/oder abhängig von einer erkannten spontanen Ein- und/oder Ausatmung des Patienten 2.

Beispielsweise kann die obere Grenze L1 jedes Mal, wenn eine spontane Einatmung als die tatsächliche Spontanatmung erkannt wird, auf einen Spitzenwert 31 gesetzt werden, der um einen vorgegebenen Betrag größer als der Referenzwert 27 ist.

Zusätzlich oder alternativ kann die obere Grenze L1 jedes Mal, wenn eine spontane Ausatmung als die tatsächliche Spontanatmung erkannt wird, auf einen Talwert 33 gesetzt werden, der gleich dem Referenzwert 27 ist. In manchen Fällen kann der Talwert 33 auch um einen vorgegebenen Betrag kleiner als der Referenzwert 27 sein. Anders ausgedrückt kann der Sollverlauf in diesem Fall abhängig von der erkannten tatsächlichen Spontanatmung abwechselnd zwischen dem Spitzenwert 31 und dem Talwert 33 (oder zwischen dem Spitzenwert 31 und dem Referenzwert 27) umgeschaltet werden.

Es ist möglich, dass die Steuereinheit 21 zwischen einem ersten Variationsmodus und einem zweiten Variationsmodus umschaltbar ist. In diesem Fall kann die obere Grenze L1 - wenn der erste Variationsmodus aktiviert ist - abhängig von einer (erkannten) Spontanatmung des Patienten 2 und - wenn der zweite Variationsmodus aktiviert ist - unabhängig von einer (erkannten) Spontanatmung des Patienten 2 variiert werden.

Beispielsweise kann die obere Grenze L1 - wenn der erste Variationsmodus aktiviert ist - zusätzlich entsprechend einer vorgegebenen Amplitude (hier einer vorgegebenen Druckdifferenz) variiert werden. Dabei kann eine Häufigkeit, mit der die obere Grenze L1 in ihrem Wert relativ zum Referenzwert 27 variiert wird, abhängig von einer Spontanatmungsfrequenz, mit der der Patient 2 spontan atmet, vorgegeben werden.

Zusätzlich oder alternativ kann die obere Grenze L1 - wenn der zweite Variationsmodus aktiviert ist - entsprechend einer vorgegebenen Frequenz und einer vorgegebenen Amplitude (hier einer vorgegebenen Druckdifferenz) variiert werden. Dabei kann eine etwaige erkannte Spontanatmung des Patienten 2 ignoriert werden.

Die vorgegebene Amplitude kann beispielsweise - je nach Zustand des Patienten 2 oder je nach Beatmungssituation - einer Druckdifferenz zwischen 2 mbar und 15 mbar, vorzugsweise von mindestens 3 mbar und/oder höchstens 10 mbar, entsprechen.

Die vorgegebene Frequenz kann beispielsweise zwischen 5 Hz und 25 Hz, vorzugsweise zwischen 10 Hz und 20 Hz, betragen. Eine periodische Variation der oberen Grenze L1 mit einer solchen Frequenz kann auch als Hochfrequenzoszillation, kurz HFO, bezeichnet werden (siehe Fig. 3).

Beispielsweise kann die obere Grenze L1 - solange (beispielsweise im ersten Variationsmodus) keine tatsächliche Spontanatmung des Patienten 2 in der jeweiligen Stabilisierungsphase S erkannt wird und/oder wenn der zweite Variationsmodus aktiviert ist - entweder entsprechend der vorgegebenen Frequenz oder entsprechend der vorgegebenen Frequenz und der vorgegebenen Amplitude variiert werden.

Der erste Variationsmodus kann beispielsweise einer Kombination eines APRV-Modus mit einem PSV-Modus in jeder Stabilisierungsphase S entsprechen.

Der zweite Variationsmodus kann beispielsweise einer Kombination eines APRV-Modus mit einem HFO-Modus in jeder Stabilisierungsphase S entsprechen.

Zweckmäßigerweise kann die Steuereinheit 21 konfiguriert sein, um einen Betrieb im zweiten Variationsmodus zu verhindern, wenn der erste Variationsmodus aktiviert ist, und/oder einen Betrieb im ersten Variationsmodus zu verhindern, wenn der zweite Variationsmodus aktiviert ist.

Die obere Grenze L1 kann beispielsweise einem positiven endexspiratorischen Druck zwischen 5 mbar und 60 mbar entsprechen. Anders ausgedrückt sollte der Istverlauf 19 beim Variieren in jeder Stabilisierungsphase S einen Druckwert von 60 mbar nicht überschreiten. Der Referenzwert 27 sollte daher um einen entsprechenden Betrag, beispielsweise um den Betrag der vorgegebenen Amplitude, niedriger als dieser maximal zulässige Druckwert gewählt werden.

Dabei kann die untere Grenze L2 - abhängig von der jeweiligen oberen Grenze L1 - beispielsweise einem positiven endexspiratorischen Druck zwischen 0 mbar und 30 mbar entsprechen. Die untere Grenze L2 kann zudem in jeder Entlastungsphase R konstant sein, d. h. während der gesamten Entlastungsphase R den gleichen Wert haben.

Die Bestimmung der vorgegebenen Frequenz und/oder der vorgegebenen Amplitude zum Variieren der oberen Grenze L1 kann auch automatisch erfolgen.

Beispielsweise kann die Sensorik 15 zu diesem Zweck einen oder mehrere Gassensoren zum Erfassen eines Kohlendioxidpartialdrucks in einer vom Patienten 2 ausgeatmeten Atemluft und/oder im Blut des Patienten 2 umfassen.

In diesem Fall können zusätzlich mittels der Sensorik 15 (genauer mittels des Gassensors oder der Gassensoren) erzeugte Partialdruckdaten 35, die einen oder mehrere (beispielsweise zeitlich aufeinanderfolgende) Partialdruckwerte des Kohlendioxidpartialdrucks umfassen, in der Steuereinheit 21 empfangen werden.

In einem weiteren optionalen Schritt können die Partialdruckdaten 35 in Kombination mit einer entsprechenden (beispielsweise im Speicher 25 als Lookup-Tabelle oder mathematische Funktion hinterlegten) Frequenzzuordnungsvorschrift, die möglichen Partialdruckwerten jeweils eine bestimmte Frequenz zuordnet, verwendet werden, um eine geeignete Frequenz für die Variation der oberen Grenze L1 auszuwählen. Die derart ausgewählte Frequenz kann dann als die vorgegebene Frequenz verwendet werden.

Zusätzlich oder alternativ können die Partialdruckdaten 35 in Kombination mit einer entsprechenden (beispielsweise im Speicher 25 als Lookup-Tabelle oder mathematische Funktion hinterlegten) Amplitudenzuordnungsvorschrift, die möglichen Partialdruckwerten jeweils eine bestimmte Amplitude zuordnet, verwendet werden, um eine geeignete Amplitude für die Variation der oberen Grenze L1 auszuwählen. Die derart ausgewählte Amplitude kann dann als die vorgegebene Amplitude verwendet werden.

Je nach Partialdruckwert kann die vorgegebene Frequenz und/oder kann die vorgegebene Amplitude auch null betragen.

Dies ermöglicht es, die Kohlendioxidabatmung in jeder Stabilisierungsphase S automatisch abhängig von einem tatsächlichen Kohlendioxidpartialdruck in der vom Patienten 2 ausgeatmeten Atemluft bzw. im Blut des Patienten 2 zu steuern.

Darüber hinaus kann die Steuereinheit 21 konfiguriert sein, um die Dauer einer jeden Entlastungsphase R abhängig von einer vom Patienten 2 in der jeweiligen Entlastungsphase R ausgeatmeten Atemluft zu steuern.

Beispielsweise können die Volumenstromdaten 29, die einen Verlauf eines Volumenstroms einer vom Patienten 2 in einer aktuellen Entlastungsphase R ausgeatmeten Atemluft anzeigen, verwendet werden, um einen Übergangszeitpunkt, zu dem die aktuelle Entlastungsphase R in eine neue Stabilisierungsphase S übergehen soll, zu bestimmen. Dabei kann der Verlauf des Volumenstroms mit einem geeigneten Schwellenwert verglichen werden und ein Zeitpunkt, zu dem der Verlauf des Volumenstroms (erstmals) den Schwellenwert erreicht, als der Übergangszeitpunkt bestimmt werden. Ein geeigneter Schwellenwert kann beispielsweise einem Anteil zwischen 10 % und 70 % oder zwischen 20 % und 40 %, vorzugsweise 25 %, eines möglichen Gesamtvolumenstroms der vom Patienten 2 bei einem einzelnen Atemzug ausgeatmeten Atemluft entsprechen.

Der Sollverlauf kann dann zum Übergangszeitpunkt von der unteren Grenze L2 auf die obere Grenze L1 umgeschaltet werden, sodass die aktuelle Entlastungsphase R in die neue Stabilisierungsphase S übergeht.

Auf diese Weise kann ein teilweiser oder vollständiger Kollaps der Lunge 13 des Patienten 2, insbesondere der Alveolen, beim Ausatmen in der jeweiligen Entlastungsphase R wirksam verhindert werden.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Beatmungsgerät
- 2: Patient
- 3: Aktorik
- 5: Atemluftstrom
- 7: Atemluftanschluss
- 11: Atemluftleitung
- 13: Lunge
- 15: Sensorik
- 17: Sensordaten
- 19: Istverlauf
- 21: Steuereinheit
- 23: Prozessor
- 25: Speicher
- 26: Steuersignal
- 27: Referenzwert
- 29: Volumenstromdaten
- 31: Spitzenwert
- 33: Talwert
- 35: Partialdruckdaten
- p: Atemluftdruck
- t: Zeit
- L1: obere Grenze
- L2: untere Grenze
- R: Entlastungsphase
- S: Stabilisierungsphase
- V: Atemluftvolumen

## Patentansprüche

1. Steuereinheit (21) für ein Beatmungsgerät (1), wobei das Beatmungsgerät (1) umfasst:
eine Aktorik (3) zum Erzeugen eines Atemluftstroms (5);
einen Atemluftanschluss (7) zum Bereitstellen des Atemluftstroms (5);
eine Sensorik (15) zum Erfassen mindestens eines Beatmungsparameters (p, V) bezüglich einer Beatmung eines Patienten (2) mit dem vom Atemluftanschluss (7) bereitgestellten Atemluftstrom (5), wobei der mindestens eine Beatmungsparameter (p, V) einen am Atemluftanschluss (7) anliegenden Atemluftdruck (p) und/oder ein vom Patienten (2) ein- und/oder ausgeatmetes Atemluftvolumen (V) umfasst;
wobei die Steuereinheit (21) konfiguriert ist, um folgendes Steuerverfahren auszuführen:
Empfangen von mittels der Sensorik (15) erzeugten Sensordaten (17), die einen Istverlauf (19) des mindestens einen Beatmungsparameters (p, V) anzeigen;
Erzeugen eines Steuersignals (26) zum Steuern der Aktorik (3) unter Verwendung der Sensordaten (17), um den Atemluftstrom (5) so anzupassen, dass sich der Istverlauf (19) einem Sollverlauf annähert, wobei der Sollverlauf mehrere Stabilisierungsphasen (S), in denen sich der Istverlauf (19) jeweils einer oberen Grenze (L1) annähern soll, und mehrere Entlastungsphasen (R), in denen sich der Istverlauf (19) jeweils einer unteren Grenze (L2) annähern soll, umfasst, wobei die Stabilisierungsphasen (S) und die Entlastungsphasen (R) einander abwechseln, wobei die obere Grenze (L1) durch einen Referenzwert (27) zum Stabilisieren der Lunge (13) des Patienten (2) und zum Ermöglichen einer Spontanatmung des Patienten (2) definiert ist und durch wiederholtes abwechselndes Erhöhen und Verringern ihres Werts relativ zum Referenzwert (27) variiert wird, um in jeder Stabilisierungsphase (S) eine zusätzliche Kohlendioxidabatmung zu bewirken, wobei die untere Grenze (L2) in ihrem Wert relativ zum Referenzwert (27) so definiert ist, dass in jeder Entlastungsphase (R) eine im Vergleich zur jeweils vorangegangenen Stabilisierungsphase (S) verstärkte Ausatmung zum Entlasten der Lunge (13) des Patienten (2) bewirkt wird.

2. Steuereinheit (21) nach Anspruch 1,
wobei das Steuerverfahren ferner umfasst:
Auswerten der Sensordaten (17), um eine tatsächliche Spontanatmung des Patienten (2) in einer der Stabilisierungsphasen (S) zu erkennen, oder
Empfangen von mittels der Sensorik (15) erzeugten Volumenstromdaten (29), die einen Verlauf eines Volumenstroms einer vom Patienten (2) ein- und/oder ausgeatmeten Atemluft anzeigen, und Auswerten der Volumenstromdaten (29) allein oder in Kombination mit den Sensordaten (17), um eine tatsächliche Spontanatmung des Patienten (2) in einer der Stabilisierungsphasen (S) zu erkennen;
wobei - wenn die tatsächliche Spontanatmung erkannt wird - die obere Grenze (L1) in der jeweiligen Stabilisierungsphase (S) abhängig von der erkannten tatsächlichen Spontanatmung variiert wird.

3. Steuereinheit (21) nach Anspruch 2,
wobei - wenn eine spontane Einatmung als die tatsächliche Spontanatmung erkannt wird - die obere Grenze (L1) auf einen Spitzenwert (31) gesetzt wird, der um einen vorgegebenen Betrag größer als der Referenzwert (27) ist, und/oder
wobei - wenn eine spontane Ausatmung als die tatsächliche Spontanatmung erkannt wird - die obere Grenze (L1) auf einen Talwert (33) gesetzt wird, der gleich dem Referenzwert (27) oder um einen vorgegebenen Betrag kleiner als der Referenzwert (27) ist.

4. Steuereinheit (21) nach einem der vorhergehenden Ansprüche,
wobei die Steuereinheit (21) zwischen einem ersten Variationsmodus und einem zweiten Variationsmodus umschaltbar ist,
wobei - wenn der erste Variationsmodus aktiviert ist - die obere Grenze (L1) abhängig von der Spontanatmung des Patienten (2) variiert wird,
wobei - wenn der zweite Variationsmodus aktiviert ist - die obere Grenze (L1) unabhängig von der Spontanatmung des Patienten (2) variiert wird.

5. Steuereinheit (21) nach einem der vorhergehenden Ansprüche,
wobei die obere Grenze (L1) entsprechend einer vorgegebenen Frequenz und/oder einer vorgegebenen Amplitude variiert wird.

6. Steuereinheit (21) nach Anspruch 5 rückbezogen auf Anspruch 4,
wobei - wenn der zweite Variationsmodus aktiviert ist - die obere Grenze (L1) zumindest entsprechend der vorgegebenen Frequenz variiert wird.

7. Steuereinheit (21) nach Anspruch 5 oder 6,
wobei das Steuerverfahren ferner umfasst:
Empfangen von mittels der Sensorik (15) erzeugten Partialdruckdaten (35), die einen oder mehrere Partialdruckwerte eines Kohlendioxidpartialdrucks in einer vom Patienten (2) ausgeatmeten Atemluft und/oder im Blut des Patienten (2) umfassen;
Bestimmen der vorgegebenen Frequenz unter Verwendung der Partialdruckdaten (35) und einer Frequenzzuordnungsvorschrift, die möglichen Partialdruckwerten jeweils eine bestimmte Frequenz zuordnet, und/oder
Bestimmen der vorgegebenen Amplitude unter Verwendung der Partialdruckdaten (35) und einer Amplitudenzuordnungsvorschrift, die möglichen Partialdruckwerten jeweils eine bestimmte Amplitude zuordnet.

8. Steuereinheit (21) nach einem der Ansprüche 5 bis 7,
wobei die vorgegebene Frequenz zwischen 5 Hz und 25 Hz, vorzugsweise zwischen 10 Hz und 20 Hz, beträgt und/oder
wobei die vorgegebene Amplitude einer Druckdifferenz zwischen 2 mbar und 15 mbar entspricht.

9. Steuereinheit (21) nach einem der vorhergehenden Ansprüche,
wobei das Steuerverfahren ferner umfasst:
Empfangen von mittels der Sensorik (15) erzeugten Volumenstromdaten (29), die einen Verlauf eines Volumenstroms einer vom Patienten (2) in einer aktuellen Entlastungsphase (R) ausgeatmeten Atemluft anzeigen;
Bestimmen eines Übergangszeitpunkts, zu dem die aktuelle Entlastungsphase (R) in eine neue Stabilisierungsphase (S) übergehen soll, unter Verwendung der Volumenstromdaten (29);
Umschalten des Sollverlaufs von der unteren Grenze (L2) auf die obere Grenze (L1) zum Übergangszeitpunkt, sodass die aktuelle Entlastungsphase (R) in die neue Stabilisierungsphase (S) übergeht.

10. Steuereinheit (21) nach Anspruch 9,
wobei der Verlauf des Volumenstroms mit einem Schwellenwert verglichen wird und ein Zeitpunkt, zu dem der Verlauf des Volumenstroms den Schwellenwert erreicht, als der Übergangszeitpunkt bestimmt wird.

11. Steuereinheit (21) nach einem der vorhergehenden Ansprüche,
wobei die obere Grenze (L1) einem positiven endexspiratorischen Druck zwischen 5 mbar und 60 mbar entspricht und/oder
wobei die untere Grenze (L2) einem positiven endexspiratorischen Druck zwischen 0 mbar und 30 mbar entspricht und/oder in jeder Entlastungsphase (R) konstant ist.

12. Steuereinheit (21) nach einem der vorhergehenden Ansprüche,
wobei ein zeitliches Verhältnis der Stabilisierungsphasen (S) zu den Entlastungsphasen (R) bezogen auf eine Referenzdauer mindestens zwei zu eins oder mindestens vier zu eins beträgt und/oder
wobei jede Stabilisierungsphase (S) mindestens zwei oder mindestens vier Atemzüge umfasst.

13. Beatmungsgerät (1), umfassend:
eine Aktorik (3) zum Erzeugen eines Atemluftstroms (5);
einen Atemluftanschluss (7) zum Bereitstellen des Atemluftstroms (5);
eine Sensorik (15) zum Erfassen mindestens eines Beatmungsparameters (p, V) bezüglich einer Beatmung eines Patienten (2) mit dem vom Atemluftanschluss (5) bereitgestellten Atemluftstrom (5), wobei der mindestens eine Beatmungsparameter (p, V) einen am Atemluftanschluss (7) anliegenden Atemluftdruck (p) und/oder ein vom Patienten (2) ein- und/oder ausgeatmetes Atemluftvolumen (V) umfasst;
eine Steuereinheit (21) nach einem der vorhergehenden Ansprüche.

14. Computerprogramm zum Betreiben des Beatmungsgeräts (1) nach Anspruch 13, wobei das Computerprogramm Befehle umfasst, die die Steuereinheit (21) beim Ausführen des Computerprogramms durch die Steuereinheit (21) veranlassen, folgendes Steuerverfahren auszuführen:
Empfangen von mittels der Sensorik (15) erzeugten Sensordaten (17), die einen Istverlauf (19) des mindestens einen Beatmungsparameters (p, V) anzeigen;
Erzeugen eines Steuersignals (26) zum Steuern der Aktorik (3) unter Verwendung der Sensordaten (17), um den Atemluftstrom (5) so anzupassen, dass sich der Istverlauf (19) einem Sollverlauf annähert, wobei der Sollverlauf mehrere Stabilisierungsphasen (S), in denen sich der Istverlauf (19) jeweils einer oberen Grenze (L1) annähern soll, und mehrere Entlastungsphasen (R), in denen sich der Istverlauf (19) jeweils einer unteren Grenze (L2) annähern soll, umfasst, wobei die Stabilisierungsphasen (S) und die Entlastungsphasen (R) einander abwechseln, wobei die obere Grenze (L1) durch einen Referenzwert (27) zum Stabilisieren der Lunge (13) des Patienten (2) und zum Ermöglichen einer Spontanatmung des Patienten (2) definiert ist und durch wiederholtes abwechselndes Erhöhen und Verringern ihres Werts relativ zum Referenzwert (27) variiert wird, um in jeder Stabilisierungsphase (S) eine zusätzliche Kohlendioxidabatmung zu bewirken, wobei die untere Grenze (L2) in ihrem Wert relativ zum Referenzwert (27) so definiert ist, dass in jeder Entlastungsphase (R) eine im Vergleich zur jeweils vorangegangenen Stabilisierungsphase (S) verstärkte Ausatmung zum Entlasten der Lunge (13) des Patienten (2) bewirkt wird.

15. Computerlesbares Medium, auf dem das Computerprogramm nach Anspruch 14 gespeichert ist.
